# EUROPEAN PATENT APPLICATION

(11) **EP 4 194 829 A1**
(43) Date of publication of application: **14.06.2023**
(21) Application number: 22466002.7
(22) Date of filing: 28.11.2022
(51) Int. Cl.: G01L 1/18, B25J 13/08, G01L 5/22, G06F 3/041

(54) **PRESSURE DISTRIBUTION SCANNING DEVICE**

(30) Priority: 08.12.2021 CZ 20210556
(71) Applicant: Ceská zemedelská univerzita v Praze, 165 00 Praha 6 (CZ)
(72) Inventor: Volf, Jaromír, 27746 V?estudy (CZ); Novák, Viktor, 25165 Ond?ejov (CZ); Papezová, Stanislava, 10000 Praha 10 (CZ); Koder, Petr, 29474 P?edm??ice nad Jizerou (CZ); Syrový, Tomás, 56501 Mostek (CZ)
(74) Representative: Jirkalova, Hana

(57) **Abstract**

The pressure distribution scanning device is characterized by consisting of tactile sensors arranged in the shape of a regular rectangular matrix. Each tactile sensor comprises an elastic non-conductive supporting foil (1) on which resistive ink (5) is applied only in places of contact with the circular electrodes (3) and (4). The resistive ink (5) may be applied directly on the concentric circular electrodes (3) and (4), which are designed as follows: The motives of the inner circular electrodes (4) of the sensor are applied on the bottom elastic supporting foil (1); a dielectric layer (2) with orifices (7) at the sites of the inner circular electrodes (3) is applied on the elastic non-conductive supporting foil (1). The motives of the outer circular electrodes (4) in the shape of concentric annuli are printed on this dielectric layer (2). The resulting structure of circular electrodes (3) and (4) in the shape of concentric annuli is then in contact with the resistive ink (5), which is applied only at the site of the circular electrodes (3) and (4). The entire structure then has circular orifices (7) cut through the interstitial space of the motives of the circular electrodes (3) and (4). On the other side of the elastic non-conductive foil (6) there is the shear layer (8) covered by an elastic protective non-conductive covering upper layer (9). This shear layer (8) has a different elasticity module in the transverse direction than the elasticity module in the longitudinal direction. The tactile sensor set designed in this way is covered on the side of the circular electrodes (3) and (4) by an elastic protective non-conductive covering bottom layer (10). Under this layer (10), a solid layer (11) may be placed to reinforce the structure, and under this a covering layer (12) may be added. Instead of the solid layer (11), a reinforced box frame (13), in which the scanner electronics can be placed, may be used.

## Description

### Technical field

The solution concerns a device for scanning tactile (touch) information, namely the distribution of pressures, and its variants in elastic execution - commonly called the plantograph.

### Prior art

Advancement in robotics, automation and non-invasive diagnostics in medicine and elsewhere is accompanied by a growing need to acquire information on the interaction of the robot with its environment, as well as information on the technological operations in progress. The scanning of contact pressures is an important characteristic of the mutual action of systems or their parts. For example, the distribution of pressure on a tyre which is in contact with the road surface, or on conveyor belts and their even tensioning. A very important requirement is the determination of pressure distribution between a living organism and its environment in biomechanics, where pathological distribution of pressures may cause serious health issues. Also, the distribution of pressures may be applied in the non-invasive diagnostics of various diseases or disorders of the human or animal skeletal-muscular systems.

Current systems commonly apply tactile sensors based on piezoresistive materials, piezoresistive foils and capacitive sensors. They usually have a lower density of individual sensors and their design does not allow long-term and impact overload. Other systems in use apply conductive elastomers. One such example is the solution according to patent CZ 295655. This is a proportion scanner of contact pressure distribution, consisting of tactile sensors. The tactile sensors are arranged in the shape of a regular rectangular matrix. Each tactile sensor comprises a core formed by a conductive elastomer foil, which has, on one side, the first band electrode and, on the other side, the second band electrode placed vertically to the first band electrode. A shear layer with a different elasticity module in the transverse direction than the elasticity module in the longitudinal direction is placed on the first band electrode. The tactile sensor set is thus covered both from the top, from the side of the shear layer, by a protective, non-conductive top covering layer, and from the bottom, from the side of the second band electrode, by a protective non-conductive bottom covering layer. These types achieve a higher density of sensors, they resist long-term and impact overload; their downside is the complex multi-layered structure of the sensor with two electrode plates on each side of the conductive elastomer.

### Nature of the patent

The above-mentioned downside of the sensor design with two layers of electrodes has been resolved by a pressure distribution scanning system consisting of tactile sensors, in which the tactile sensors are arranged in the shape of a regular rectangular matrix, each tactile sensor comprising a layer of resistive ink that comes into contact with the electrodes where, according to the invention, the resistive ink is in contact on the elastic non-conductive foil with the inner circular electrode and outer circular electrode, of which the inner circular electrode is applied on the elastic non-conductive supporting foil and the outer circular electrode is applied concentrically in the shape of an annulus on the dielectric layer; orifices are cut through the interstitial space between the circular electrodes.

The device according to the invention is characterised by the application of resistive ink directly on the electrodes and the device covered from the top by an elastic non-conductive foil.

The device according to the invention is also characterised by tactile sensors arranged in the shape of a regular rectangular matrix with each tactile sensor comprising a layer of resistive ink that comes into contact with the electrodes, a shear layer that has a different elasticity module in the transverse direction than the elasticity module in the longitudinal direction, and an elastic protective non-conductive covering upper and lower layer, where the elastic protective non-conductive upper layer covers the shear layer and the resistive ink is applie3d on the non-conductive elastic foil or directly on the electrodes and is in contact with the inner circular electrode and outer circular electrode, of which the inner circular electrode is applied on the elastic non-conductive foil and the outer circular electrode is applied concentrically in the shape of an annulus on the dielectric layer, the shear layer covered by an elastic protective non-conductive covering upper layer is located on the other side of the elastic non-conductive foil or on the resistive ink, and the elastic protective non-conductive covering bottom layer covers the tactile sensor set composed in this way from the bottom side of the elastic non-conductive supporting foil.

The device according to the invention is characterized by orifices through the elastic non-conductive supporting foil, a dielectric layer and elastic non-conductive layer in the interstitial space between the inner circular electrode and outer circular electrode and in the interstitial space of the resistive ink.

The device according to the invention is further characterised by the inner circular electrode and outer external circular electrode created on an elastic non-conductive supporting foil and dielectric layer.

The device according to the invention is also characterised by a solid plate attached to the elastic protective non-conductive covering bottom layer.

The device according to the invention is also characterised by a reinforced box frame structure attached to the elastic protective non-conductive covering bottom layer.

The device according to the invention is also characterised by the inner circular electrode and outer circular electrode set on the elastic non-conductive supporting foil and dielectric layer, where the elastic non-conductive supporting layer is solid.

The device according to the invention is also characterised by that the inner circular electrode and outer circular electrode are created on the elastic non-conductive supporting foil and elastic dielectric layer.

The device according to the invention is also characterised by that the solid plate or reinforced box frame structure are covered by an elastic protective non-conductive covering layer.

The device according to the invention is also characterised by the insertion of an antistatic layer between the elastic protective non-conductive covering bottom layer and the solid plate, or the solid plate itself is the antistatic layer

The device according to the invention is also characterised by the sandwich structure of the shear layer.

The device according to the invention is also characterised by the elastic protective non-conductive covering upper layer and/or the elastic protective non-conductive bottom layer and /or the elastic protective non-conductive made of antistatic materials.

The main part of the tactile information scanning device according to the invention are the tactile sensors. A sensor can be executed as solid or elastic. Each tactile sensor consists of a layer of conductive ink with an elastic plate on top, which covers the layer of conductive ink that is in contact with the circular electrodes set. The latter have a multi-layered structure. The motives of the inner electrodes of the sensor are applied on the bottom elastic layer on which a dielectric layer with orifices at the sites of the inner electrodes is applied. On this dielectric layer the motives of the outer circular electrodes in the shape of an annulus are printed. The structure of the circular electrodes in the shape of an annulus created in this way is in contact with the resistive ink applied only at the site of the electrodes. Orifices, namely circular orifices, are then cut through the entire structure in the interstitial space of the motives of the circular electrodes to enhance the elasticity of the whole scanner. A shear layer which in the transverse direction has a different elasticity module than the elasticity module in the longitudinal direction may be placed on the layer with the resistive ink. The tactile sensor set created in this way can be covered from both the top, from the side of the shear layer, by a top elastic protective non-conductive covering layer, and from the bottom, from the side of the electrode foil, by a bottom elastic protective non-conductive covering layer.

A different execution has an elastic intermediate layer inserted between the bottom elastic protective non-conductive covering layer and the tactile sensor set, which has the advantage of better protection of the sensor against impact overload.

In the non-elastic execution of the sensor, each tactile sensor comprises the basic structure of the scanner with, on one side, a solid plate with printed layers of the motive of the electrodes. A shear layer, which in the transverse direction has a different elasticity module than the elasticity module in the longitudinal direction, may be placed on the top layer of the scanner structure with the resistive ink. The tactile sensor set designed in this way is covered from both the top, from the side of the shear layer, by an elastic protective non-conductive covering upper layer, and from the bottom, from the side of the plate of the electrodes, by an elastic protective non-conductive covering bottom layer. From the side of the solid plate of the electrodes, the tactile sensor is equipped with the first elastic non-conductive layer to which a solid plate or reinforcement box is attached. The box is located under the scanner set and contains the scanner electronics.

One possible execution has a solid plate, or reinforced box frame structure, covered by an elastic protective non-conductive covering layer; the advantage is enhanced protection against impact overload.

A convenient execution of any of the mentioned variants involves a sandwich structure of the shear layer. It is also convenient in both variants if an elastic protective non-conductive covering upper layer, and/or elastic protective non-conductive covering bottom layer, and/or an elastic protective non-conductive covering layer of antistatic layer between the elastic protective non-conductive covering bottom layer and the tactile sensor set is placed. This will allow enhanced resistance to long-term and impact overload and enhanced protection against interference.

The benefit of the described sensor is its simpler structure compared to currently used sensors with two layers of electrodes; the sensor is also resistant to overload and impact forces generated during dynamic operations. It offers a simpler structure, greater density of sensors and the achievement of up to 25 times greater impact overload. It can be expected to meet the requirements also of operations in difficult environments. The device according to the invention provides not only information that the object of the surrounding environment is in contact with the sensor, but also information about the point of contact, pressure, and its distribution on the area of the scanner. Where forces that used to result in deformation are exceeded, the structure presents effective protection of the scanner sensor itself. Moreover, the sensor does not contain any mobile mechanical element.

The advantage of the device according to the invention is that it allows a large scope of measurement of pressure distribution and with a high density of sensors, where the individual sensors of the scanner are protected against overload and the acting pressure is transferred to an electric signal via the resistive ink applied on the circular electrodes.

### List of figures in the drawings

The device according to the invention is explained in the figures on the attached drawings, where Fig. 1 presents a sectional view of the actual solution of the elastic scanner for scanning pressure distribution in the device according to the invention, and Fig. 2 is a schematic view of the execution of the circular electrodes of the device according to the invention for scanning pressure distribution. Fig. 3 is a schematic sectional view of the execution of the possible layout of the whole device according to the invention for scanning pressure distribution.

The device according to the invention has been successfully tested in practice by the inventors at the laboratories of the applicant, which is the Czech University of Life Sciences in Prague (Česká zemědělská univerzita v Praze, CZU), Faculty of Engineering (Technicka fakulta), Kamýcká 129, 165 21 Praha 6 - Suchdol, CZ.

### Examples of the performance of the invention

### Example 1

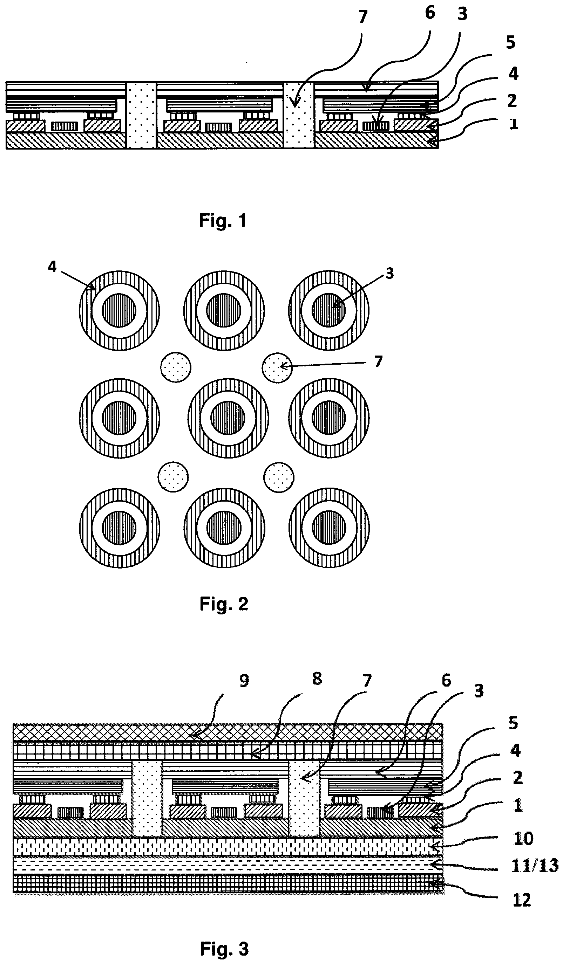

The execution according to Fig. 1 refers to the device according to the invention for scanning the distribution of pressures with a sandwich structure. The top layer is an elastic non-conductive foil **6** with applied resistive ink **5** in the place of contact between the resistive ink and circular electrodes **3** and **4.** Electrodes **3** and **4** may have a different shape, e.g., angular, or oval or spiral, but the circular shape has proven to be the most convenient. Resistive ink **5** can be applied directly on the circular electrodes **3** and **4.** The resistive ink **5** changes its resistance under the effect of the pressure, thus creating a pressure - electric signal converter. The thickness of the resistive ink **5** is not critical; in fact, it is in units or tens of µm and may be thicker. The span of the resistance of the sensor and partly also its sensitivity depends on the thickness. The presented example used resistive ink **5** from various manufacturers, e.g., Loctite - Henkel. The resistive ink 5 is applied on the elastic non-conductive foil **6** opposite the motive of the circular electrodes **3** and **4.** It can also be applied directly on the circular electrodes **3** and **4,** which are designed in the following way: The motives of the inner circular electrodes **3** of the sensor are applied on the bottom elastic non-conductive layer **1;** the dielectric layer **2** with orifices **7** at the site of the external circular electrodes **4** is applied on the elastic non-conductive supporting foil 1. The motives of the external circular electrodes **4** in the shape of an annulus are printed on this dielectric layer 2. The structure of circular electrodes **3** and **4** in the shape of an annulus created in this way is then in contact with the resistive ink **5** applied only at the site of both circular electrodes **3** and **4.** Circular orifices **7** are then cut through the whole structure in the interstitial space of the motives of the circular electrodes 3_and **4,** see Fig. 2, to enhance the elasticity of the entire device according to the invention. The motives of the circular electrodes **3** and 4 are printed with silver ink directly on to the elastic non-conductive supporting foil **1** and dielectric layer **2** according to the above-described procedure. A different resistance material may be used. The circular electrodes **3** and **4** can be metal coated - preferably gilded - to prevent oxidation.

The description of the device according to the invention in Fig. 1 describes the basic structure of the scanner in which it can be used to measure the distribution of pressures. In this case, however, it has no protection against overload or damage.

To improve the properties of the device according to the invention - e.g. the transfer of pressures to the scanner proper - an important layer of the sensor is shear layer **8,** which must have a different elasticity module in the transverse and longitudinal directions, thus enabling correct transfer of pressure distribution between the object and the sensor. This shear layer 8, which can have, for example, a sandwich structure, deals with the marginal effect and allows uniform distribution of pressure.

Protection of the sensor against damage is ensured from the top and bottom side of the sandwich structure, and that by an elastic protective non-conductive covering top layer **9,** made of, for example, PVC foil or rubber foil, placed on the shear layer **8,** and by an elastic protective non-conductive covering bottom layer 10, with an antistatic advantage, having the same properties as the elastic protective non-conductive covering upper layer **9,** located under the basic elastic non-conductive supporting foil **1.** The structure can also be designed with an independent intermediate layer, preferably antistatic (not depicted in Fig. 2), between the elastic protective non-conductive covering bottom layer **10** and the basic elastic non-conductive supporting foil **1.** The elastic protective non-conductive covering upper layer **9** and the elastic protective non-conductive covering bottom layer 10 must be resistant to the water and solvents used in washing and disinfection etc, and at the same time must serve as an insulant.

The scanner designed in this way serves to measure the distribution of pressure on a loaded surface. The object acts through the elastic protective non-conductive covering upper layer **9** and shear layer **8** on the sensor itself, which is formed by resistive ink **5** and circular electrodes **3** and **4** placed on its bottom side on the elastic non-conductive supporting foil **1** and non-conductive dielectric layer **2.** Correct application of the pressure is ensured by shear layer **8,** which must have different elasticity modules in the transverse and longitudinal directions. Resistive ink **5** is thus deformed by the applied pressure end changes its resistance in dependence on the magnitude of the pressure. Changes of this pressure are then scanned by the circular electrodes 3 and **4.** Protection of the sensor itself against permanent and impact overload is ensured by the elastic protective non-conductive bottom layer 10.

The benefit of this elastic sensor is that it can trace the effect of the unevenness of the base on the pressure applied by the loading object, for example the distribution of pressure between the seat of a chair, wheelchair etc., and the seat part of a sitting person's body.

A similar procedure can be used to make a tactile information scanning device that is not elastic. This is in essence an analogical setup where the tactile information scanning device according to the invention is formed by a sandwich structure according to Fig. 3. The heart of this tactile information scanning device is, once again, a layer of resistive **ink 5,** which changes its resistance according to the applied pressure, thus forming a pressure - electric signal converter. The resistive ink **5** is placed on one side of the elastic non-conductive foil **6** and is in contact with the annular-shaped circular electrodes **3** and **4** created on the elastic non-conductive supporting foil **1** and the non-conductive dielectric layer **2** and on the other side by shear layer **8.** Circular electrodes **3** and **4** are designed as follows: Motives of the inner circular electrodes **3** of the sensors are printed on the bottom elastic non-conductive supporting foil **1;** dielectric layer **2** with orifices **7** is applied at the sites of the inner circular electrodes 3. Motives of the annular external circular electrodes **4** are printed on this dielectric layer **2.** The thus created structure of the circular electrodes **3** and **4** in the shape of an annulus is then in contact with the resistive ink **5** applied on the elastic non-conductive foil **6** only at the site of the circular electrodes **3** and **4.** The whole structure has circular orifices **7** cut through the interstitial space of the motives of the circular electrodes **3** and **4** (see Fig. 2) to enhance the elasticity of the whole scanner. In the non-elastic execution of the device according to the invention this cutting through need not be done. This allows greater density of the sensors, i.e., smaller diameters of the circular electrodes **3** and **4.** The motives of the circular electrodes **3** and **4** are printed with silver resistive ink directly on the bottom side of the elastic non-conductive supporting foil **1** and dielectric layer **2** according to the above-described procedure. A different resistive material may be used. The circular electrodes **3** and **4** may be metal-coated, preferably gilded, to prevent oxidation. The non-conductive supporting foil **1** may be elastic or solid, although in this case only solid electrodes make sense, as the elastic ones are more expensive and there is no sense in using them since the whole set is solid anyway. Protection of the sensor against damage is ensured by the elastic protective non-conductive covering upper layer **9.** The effect of the unevenness of the base on the sensor is ruled out by the placement of a solid (e.g., Dural) plate **11** on the bottom side of the sensor, i.e., from the side of the elastic non-conductive supporting foil **1,** which in the given case is covered from the top by the elastic protective non-conductive covering layer 10 and from the bottom by another elastic, preferably antistatic, protective non-conductive covering layer 12. This additional elastic protective non-conductive covering layer **12** is not mandatory and the sensor need not have it. The reinforced box frame **13** or other reinforcement structure may substitute the solid plate **11.** The reinforced box frame **13** is usually placed under the scanner set; the scanner electronics can be placed inside the reinforced box frame **13.** An important layer of the scanner is the shear layer **8,** which must have a different elasticity module in the transverse and longitudinal directions, thus allowing correct transfer of pressure distribution between the object and sensor. This shear layer **8** can have, for example, a sandwich structure.

The scanner designed in this way also serves to measure the distribution of pressure on a loaded surface. The function of the scanner is the same as in the case of the elastic type, with the difference that the effect of the unevenness of the base on which the whole contact pressure distribution sensor is placed is compensated by the solid plate **11.** Protection of the sensor proper against permanent and impact overload is provided by the elastic protective non-conductive covering bottom layer 10 and the elastic protective non-conductive covering layer **12,** which are preferably antistatic. The elastic scanner is a structure consisting of elastic layers, which make it fully flexible. It has no solid layer.

### Industrial applicability

The tactile information scanning device can be applied in the fields of medical orthopaedics and biomechanics to study the distribution of pressure on the sole of the foot and its dynamic changes when taking steps. The determination of the distribution of pressures on the soles of feet and their time course provides valuable information in the non-invasive diagnostics of the impairment of motor skills, orthopaedic defects, and many diseases, in the prevention of pathological pressures on the human body and resulting bedsores, e.g., the intelligent bed. In stabilimetry, the described sensors can be used to measure body sway; in physiotherapy, they can be used in rehabilitation to develop rehabilitation aids and prostheses and in biological feedback. The elastic variant of the device can be used to measure pressures in prosthetic devices and for the best possible adaptation of the stump of the limb, and to design seats for paraplegics and for preventive feedback. It can also be applied in designing anatomical shapes of seats and backrests, especially in the automotive and aircraft industries. It can be used also in sport medicine and methodology, in robotics to stabilise and balance robots, to determine the point of firm grip, for force measurement, etc., and in other industrial applications where it is important to know the distribution of pressures, e.g., tyre - road surface.

### List of reference characters

- 1 -: elastic non-conductive supporting foil
- 2 -: dielectric layer
- 3 -: inner circular electrode
- 4 -: outer circular electrode
- 5 -: resistive ink
- 6 -: elastic non-conductive foil
- 7 -: orifice
- 8 -: shear layer
- 9 -: upper layer
- 10 -: bottom layer
- 11 -: solid plate
- 12 -: covering layer
- 13 -: reinforced box frame

## Claims

1. The pressure distribution scanning device with tactile sensors arranged in the shape of a regular rectangular matrix and each tactile sensor consisting of a layer of resistive ink that comes into contact with the electrodes, **characterised in that** the resistive ink (5) on an elastic non-conductive foil (6) is in contact with the inner circular electrode (3) and the outer circular electrode (4), of which the inner circular electrode (3) is applied on an elastic non-conductive supporting foil (1) and the outer circular electrode (4) is applied concentrically in the shape of an annulus on a dielectric layer (2), and **in that** orifices (7) are placed in the interstitial space of the circular electrodes (3) and (4).

2. The device according to claim 1, **characterised in that** the resistive ink (5) is applied on electrodes (3) and (4) directly and the device is covered from the top by an elastic non-conductive foil (6).

3. The device according to claims 1 and 2, **characterised in that** the tactile sensors are arranged in the shape of a regular rectangular matrix, with each tactile sensor consisting of a layer of resistive ink (5) that comes into contact with the electrodes, the shear layer (8), which has a different elasticity module in the transverse direction than the elasticity module in the longitudinal direction, the elastic protective non-conductive covering upper (9) and bottom layers (10), where the elastic protective non-conductive covering upper layer (9) covers the shear layer (8) and the resistive ink (5) is applied on the non-conductive elastic foil (6) or directly on the electrodes and is in contact with the inner circular electrode (3) and the outer circular electrode (4), of which the inner circular electrode (3) is applied on the elastic non-conductive foil (1) and the outer circular electrode (4) concentrically in the shape of an annulus on the dielectric layer (2); the shear layer (8) covered by an elastic protective non-conductive covering upper layer (9) is placed on the other side of the elastic non-conductive foil (6) or on resistive ink (5), with the elastic protective non-conductive covering bottom layer (10) covering the thus created tactile sensor set from the bottom part of the elastic non-conductive supporting foil (1).

4. The device according to claims 1 and 3, **characterised in that** it is equipped with orifices (7) through the elastic non-conductive supporting foil (1), the dielectric layer (2) and elastic non-conductive foil (6) in the interstitial space between the inner circular electrode (3) and outer circular electrode (4) and in the interstitial space of the resistive ink (5).

5. The device according to claims 1 to 4, **characterised in that** a solid plate (11) is attached to the elastic protective non-conductive covering bottom layer (10).

6. The device according to claims 1 to 5, **characterised in that** a reinforced box frame structure (13) is attached to the elastic protective non-conductive covering bottom layer (10).

7. The device according to claims 1 to 6, **characterised in that** the elastic non-conductive carrier foil (1) is a solid foil.

8. The device according to claims 1 to 7, **characterised in that** the dielectric layer (2) is elastic.

9. The device according to claims 6 to 8, **characterised in that** the solid plate (11) or the reinforced box frame (13) are covered by an elastic protective non-conductive covering layer (12).

10. The device according to claims 1 to 9, **characterised in that** an antistatic layer is inserted between the elastic protective non-conductive covering bottom layer (10) and the solid plate (11), or the solid plate (11) itself is the antistatic layer.

11. The device according to claims 1 to 10, **characterised in that** the shear layer (8) is a sandwich structure.

12. The device according to claims 1 to 11, **characterised in that** the elastic protective non-conductive upper covering layer (9) and/or elastic protective non-conductive covering bottom (10) and/or elastic protective non-conductive solid plate (11) and/or elastic protective non-conductive covering layer (12) are antistatic materials.
